# EUROPÄISCHE PATENTSCHRIFT

(11) **EP 3 281 653 B1**
(45) Veröffentlichungstag und Bekanntmachung des Hinweises auf die Patenterteilung: **14.04.2021**
(21) Anmeldenummer: 17184560.5
(22) Anmeldetag: 02.08.2017
(51) Int. Cl.: A61M 1/10

(54) **PERISTALTIKPUMPE MIT MODULAREM GEHÄUSE**
PERISTALTIC PUMP WITH MODULAR HOUSING
POMPE PÉRISTALTIQUE COMPRENANT UN BOÎTIER MODULAIRE

(30) Priorität: 11.08.2016 DE 102016114958
(43) Veröffentlichungstag der Anmeldung: 14.02.2018
(73) Patentinhaber: B. Braun Avitum AG, 34212 Melsungen (DE)
(72) Erfinder: SCHÄFER, Oliver, 36286 Neuenstein (DE)
(74) Vertreter: Winter, Brandl - Partnerschaft mbB

(56) Entgegenhaltungen:
- WO-A1-2010/090944
- WO-A1-2010/093946
- WO-A2-97/10436
- US-A- 5 044 902
- US-A1- 2010 129 248

## Beschreibung

### Hintergrund der Erfindung

Die Erfindung betrifft eine Peristaltikpumpe für eine Vorrichtung zur extrakorporalen Blutbehandlung, insbesondere für eine Dialysemaschine, zum Fördern von Fluid in der Vorrichtung bzw. in der Dialysemaschine, welche Peristaltikpumpe einen um eine Rotorachse rotierbaren Rotor und ein den Rotor zumindest teilweise umgebendes Pumpengehäuse mit einer um die Rotorachse bogenförmig ausgebildeten Stützfläche aufweist, wobei eine elastisch verformbare Fluidleitung zwischen dem Rotor und der Stützfläche positionierbar ist bzw. positioniert ist und bei zur Fluidförderung durch Rotation des Rotors eine Querschnittsverengung ausbildend zwischen diesem und der Stützfläche verformt wird.

### Stand der Technik

Bekannte Peristaltikpumpen bei Medizingeräten zur extrakorporalen Blutbehandlung bestehen in der Regel aus einem Rotor, einem Pumpengehäuse und einer zwischen Rotor und Pumpengehäuse angeordneten elastischen Schlauchleitung als Fluidleitung. Das Pumpengehäuse ist in der Regel einteilig ausgebildet und bildet neben anderen Funktionen wie zum Beispiel einem Ausbilden von Fluidleitungen und Anschlüssen, Halterungen für Sensoren und Abdeckung/Abschirmung bestimmter Bestandteile der Pumpe gegenüber der Umwelt eine Stützfläche für die Fluidleitung aus. Aus dem Stand der Technik sind aufgesetzte Blutpumpengehäuse bekannt, zum Beispiel als separates Aluminium-Frästeil oder Kunststoff-Spritzgussteil ausgebildet und auf eine Gehäusefront des Gerätes montiert (siehe z.B. WO97/10436).

Des Weiteren sind aus dem Stand der Technik mehrteilige Pumpengehäuse bekannt, beispielsweise aus der WO 2012/162512 A1 oder aus der EP 2 397 695 A1, die eine Rohrpumpe offenbart, umfassend ein Gehäuse mit einer zylindrischen Seitenwand in einem Innenabschnitt davon sowie einen mit dem Gehäuse koppelbaren Halter für Verbindungsabschnitte für Fluidleitungen. Die Pumpe umfasst des Weiteren einen Rotor mit einer Walze, die auf einer Innenseite der zylindrischen Seitenwand des Gehäuses angeordnet ist, wobei der Rotor durch eine Antriebsquelle gedreht wird. Die Rohrpumpe umfasst eine Leitung mit einem gekrümmten Abschnitt zur Anbringung zwischen der zylindrischen Seitenwand und der Walze. Die Rohrpumpe ist konfiguriert, den Rotor zu drehen, um den gekrümmten Abschnitt über die Walze zusammen zu quetschen, wodurch ein Fluid durch die Leitung gepumpt wird, wobei zuflussseitig und abflussseitig ein erster Verbindungsabschnitt vorgesehen ist. Der Halter weist zweite Verbindungsabschnitte zum Verbinden mit den ersten Verbindungsabschnitten der Leitung auf, wobei die zweiten Verbindungsabschnitte mit den ersten Verbindungsabschnitten der Leitung in Eingriff stehen, um die Leitung zu halten. Derartige mehrteilige Pumpengehäuse bestehen zwar aus mehreren miteinander gekoppelten und verbundenen Teilen, diese sind aber nicht gegen ähnliche, aber nicht identische Teile austauschbar. Insbesondere ist kein modulweises Konzept mehrteiliger Pumpengehäuse bekannt, bei dem unterschiedliche Module ausgetauscht und mit anderen kombiniert und gegen andere ersetzt werden können. Man kann auch sagen, dass es aus dem Stand der Technik nicht bekannt ist, eine Einheitlichkeit an Komponenten selbst bei variierenden Gehäuseformen zu realisieren.

Aus der DE 10 2010 000 594 A1 ist bekannt eine Schlauchpumpe zur Förderung eines in einem Schlauch geführten Mediums mit mehreren Quetschelementen, die den Schlauch unter Quetschung gegen ein Gegenlager drücken und dadurch das Medium in dem Schlauch in Förderrichtung fördern. Um bei einer derartigen Schlauchpumpe eine einfache und schnelle Einführung des Schlauchs zu ermöglichen, verfügt die Schlauchpumpe über eine Einfädeleinrichtung zum Einführen des Schlauchs zwischen die Quetschelemente und das Gegenlager.

Aus der DE 10 2012 104 461 A1 ist bekannt ein medizinisches Gerät zur extrakorporalen Blutbehandlung, umfassend einen extrakorporalen Blutkreislauf mit einem Schlauch und zwei auf dem Schlauch messende Sensoreinheiten mit unterschiedlichen Wirkprinzipien, an welche Abschnitte des Schlauchs für eine Messung ankoppelbar sind, wobei jede Sensoreinheit aus einem sensorspezifischen Bauteil und einem sensorneutralen Bauteil besteht, wobei die sensorneutralen Bauteile der Sensoreinheiten identisch ausgebildet sind, während sich die sensorspezifischen Bauteile abhängig vom Wirkprinzip unterscheiden und jeweils eine spezifische Sensorik umfassen, und dass für jede Sensoreinheit ein sensorneutrales Bauteil auf einem sensorspezifischen Bauteil montiert ist, wobei ein Abschnitt des Schlauchs bei Einbringung und Fixierung in einem sensorneutralen Bauteil so an die Sensorik des darunter liegenden sensorspezifischen Bauteils ankoppelbar ist, dass mit der Sensorik eine Messung am Schlauch durchführbar ist.

Bei bekannten Systemen ist es von Nachteil, dass unterschiedliche Funktionselemente oder -geometrien des Gehäuses durch ein einziges oder mit einem einzigen Bauteil, insbesondere in Form eines einteiligen Gehäuses, realisiert sind. Man kann auch sagen, dass bekannte Gehäuse eine Mehrzahl an Funktionen zu erfüllen haben, wie zum Beispiel eine Ausbildung der Stütz- oder Laufläche zusammen mit dem Ausbilden eines gewissen äußeren Design oder Erscheinungsbilds der Pumpe oder Haltefunktionen für Zu- und Ableitungen oder simple Abdeckfunktionen. Dadurch ist die Verwendung bestehender Pumpen in anderen Gerätevarianten aufgrund eines feststehenden äußeren Design nicht oder nur bedingt möglich. Die Verwendung einer bestimmten bekannten Pumpe mit einer bestimmten Pumpencharakteristik, die im Wesentlichen durch Parameter der Stützfläche bestimmt sein kann, mit einer anderen Abdeckung oder an einer anderen Maschine mit unterschiedlichen Anschlussmöglichkeiten der Pumpe ist in der Regel unmöglich.

### Kurzbeschreibung der Erfindung

Ausgehend von dem vorstehend beschriebenen Stand der Technik liegt der vorliegenden Erfindung die Aufgabe zugrunde, die zuvor angeführten Nachteile zu beseitigen, insbesondere eine Peristaltikpumpe für eine Vorrichtung zur extrakorporalen Blutbehandlung, insbesondere für eine Dialysemaschine, bereit zu stellen, die eine höhere Variabilität des äußeren Pumpendesign bei gleichbleibender Funktionsstruktur und/oder eine höhere Variabilität hinsichtlich der Pumpencharakteristik bei unverändertem Pumpendesign und geringem Entwicklungsaufwand ermöglicht.

Nach der Erfindung, wie beansprucht, wird diese Aufgabe gelöst durch eine Peristaltikpumpe für eine Vorrichtung zur extrakorporalen Blutbehandlung, insbesondere für eine/einer Dialysemaschine, zum Fördern von Fluid in der Vorrichtung, wobei die Peristaltikpumpe einen um eine Rotorachse rotierbar angetriebenen Rotor und ein den Rotor zumindest teilweise umgebendes Pumpengehäuse mit einer um die Rotorachse bogenförmig ausgebildeten Stützfläche aufweist, die an einer hufeisenförmig gebogenen Innenwandung ausgebildet ist, die über Verstrebungen mit einer ebenfalls gebogenen Außenwandung verbunden ist, wobei eine elastisch verformbare Fluidleitung zwischen dem Rotor und der Stützfläche positionierbar (entfernbar positioniert) ist und bei zur Fluidförderung durch Rotation des Rotors eine Querschnittsverengung ausbildend zwischen diesem und der Stützfläche (fortlaufend) verformt wird, wobei das Pumpengehäuse derart modular ausgebildet ist, indem es ein die Stützfläche ausbildendes Stützflächenmodul und ein mit diesem zu koppelndes und den Rotor zumindest teilweise umgebendes Gehäuse-/Abdeckungsmodul aufweist.

Allgemeiner ausgedrückt hat das Pumpengehäuse zumindest zwei miteinander koppelbare Gehäusemodule, nämlich ein erstes Modul, das für das Umgreifen bzw. Einschließen/Aufnehmen des Rotors angepasst ist und ein zweiten Modul, das für das Bereitstellen einer Stützfläche für eine darin eingelegte flexible/quetschbare Fluidleitung angepasst ist, gegen welche die Fluidleitung mittels des Rotors unter Ausbildung von Fluidaufnahmekammern in der Fluidleitung andrückbar ist. Auf diese Weise kann das erste Modul unabhängig vom zweiten Modul designt werden, wobei unterschiedlich designte erste Module beliebig mit dem zweiten Modul kombinierbar sind.

Die Aufgabe wird außerdem gelöst durch eine Vorrichtung zur extrakorporalen Blutbehandlung (extrakorporale Blutbehandlungsmaschine/Dialysemaschine) mit einer erfindungsgemäßen Peristaltikpumpe, insbesondere wie in der vorliegenden Beschreibung offenbart oder wie in den angehängten Ansprüchen beansprucht ist.

Die Erfindung betrifft also ein modulartig aufgebautes Gehäuse einer Blutpumpe, insbesondere einer Peristaltikpumpe, wobei die Funktionen des Gehäuses in unterschiedliche Funktionsmodule aufgetrennt sind. Die unterschiedlichen Module nach der Erfindung können insbesondere sein
- das Stützflächenmodul, (Hauptfunktionsmodul Lauffläche), das mit seinem zylindrischen Radius und seinem Umschlingungswinkel der Stützfläche das Fördervolumen mitbestimmt, sowie
- das Gehäusemodul (Funktionsmodul Sichtblende oder Designabdeckung), das das äußere Erscheinungsbild der Pumpe definiert.

Das Gehäusemodul kann weitere Funktionen realisieren und Funktionselemente aufweisen, wie zum Beispiel
- eine Schlauchaufnahme ausbilden,
- Sensoren aufnehmen und umhüllen,
- Deckelaufnahmen beinhalten, etc..
Das Gehäusemodul kann insbesondere dazu dienen, ein Überleitsystem, zum Beispiel in Form der elastisch verformbaren Fluidleitung zu führen und/oder zu halten. Die einzelnen Module können insbesondere zu einem Satz an entsprechenden Modulen gehören.

Die Peristaltikpumpe der erfindungsgemäßen Vorrichtung kann in üblicher Weise ein definiertes Volumen eines Mediums fördern, wie zum Beispiel Blut oder Dialysefluid, von einer Niederdruckseite, in der Regel die arterielle Seite, auf eine Hochdruckseite, in der Regel die venöse Seite. Die elastische Fluidleitung wird in diese zwischen Rotor und der durch das Stützflächenmodul ausgebildeten Stützfläche schlaufenförmig eingelegt. Sie kann dabei insbesondere durch das Gehäusemodul geführt oder gehalten sein. Der Rotor und die die elastische Fluidleitung stützende Stützfläche sind derart ausgebildet und aufeinander abgestimmt, dass zwischen ihnen eine Förderstrecke ausgebildet ist. In deren Verlauf kommt es bei Rotation des Rotors um die Rotorachse zu Verformen und Abklemmen der elastisch verformbaren Fluidleitung. Der Rotor ist derart ausgebildet, dass die Fluidleitung nur punktuell oder abschnittsweise zusammengequetscht wird. Er kann zum Beispiel gegen die Fluidleitung vorgespannte und/oder gegenüber der Rotorachse relativpositionierbare Quetschelemente aufweisen. Die durch den Kontakt mit dem Rotor verursachte Quetschstelle wandert mit dem rotierenden Rotor mit und wird quasi durch die Fluidleitung von der Niederdruckseite zur Hochdruckseite bewegt. Infolge dessen wird Fluid in Förderrichtung aus der Fluidleitung herausgedrückt. Nachfließendes Fluid wird durch Niederdruck, insbesondere Vakuum, der aufgrund elastischer Rückformung der Fluidleitung nach der Verformung durch den Rotor entsteht, in die Leitung hineingezogen. Die elastisch verformbare Fluidleitung kann zum Beispiel ein Schlauch sein.

Mit der Erfindung können insbesondere die folgenden Vorteile erzielt werden:
- Durch Einsatz der erfindungsgemäßen grundsätzlichen Trennung von Funktionen durch Vorsehen mehrerer Funktionsmodule kann im konkreten ermöglicht werden, dass Hauptfunktionsmodule einer Blutpumpe, die die Stützoder Laufflächen ausbilden oder beinhalten, nur einmalig entwickelt und qualifiziert werden müssen (universal einsetzbar) und im weiteren Einsatz über standardisierte Schnittstellen mit anderen, unterschiedlichen Funktionsmodulen, wie einem oder mehreren Gehäusemodulen und/oder einem oder mehreren Montagemodulen (individuell einsetzbar) kombiniert werden können.
- Dies ermöglicht die mehrfache Verwendung der unterschiedlichen Module in unterschiedlichen Kombinationen an verschiedenen Maschinen unter unterschiedlichen Randbedingungen. Entwicklungszeiten und Qualifizierungsaufwand können in vorteilhafter Weise verringert werden.
- Durch die Splittung von Funktionen und deren Zuordnung in separate Funktionsmodule ist eine hohe Variation bei funktionskritischen Gleichteilen möglich.
- Es können mehr Varianten mit identischen Gleichteilen gefertigt werden, was zu einer Kostensenkung bei den Gerätevarianten führt.

Vorteilhafte Ausführungsformen der Erfindung sind in den Unteransprüchen beansprucht und werden nachfolgend näher erläutert.

Eine Ausführungsform der Peristaltikpumpe ist dadurch gekennzeichnet, dass das Pumpengehäuse des Weiteren ein Montagemodul aufweist. Dieses kann entweder als separates Bauteil ausgeführt sein oder Bestandteil eines Gehäuses einer Vorrichtung zur extrakorporalen Blutbehandlung, insbesondere einer Gerätefront, sein. Das Montagemodul kann insbesondere einerseits mit dem Stützflächenmodul und/oder mit dem Gehäusemodul koppelbar bzw. gekoppelt sein. Andererseits kann es, auch unmittelbar, mit der Vorrichtung zur extrakorporalen Blutbehandlung, insbesondere mit dessen Gehäuse koppelbar sein oder gekoppelt sein.

Vorzugsweise kann das Stützflächenmodul zumindest eine standardisierte Schnittstelle zum Koppeln mit dem Gehäusemodul und/oder dem Montagemodul und/oder mit einem Gehäuseelement der Vorrichtung aufweisen. Koppeln in diesem Sinne umfasst zum Beispiel ein Anordnen, Festlegen oder Befestigen der genannten Module miteinander oder aneinander. Es ist von besonderem Vorteil, wenn die Peristaltikpumpe nach einer Ausführungsform ein Gehäusemodul mit einer standardisierten Schnittstelle zum Koppeln mit dem Stützflächenmodul und/oder dem Montagemodul und/oder mit einem Gehäuseelement der Vorrichtung aufweist. Auch kann das Montagemodul eine standardisierte Schnittstelle zum Koppeln mit dem Stützflächenmodul und/oder dem Gehäusemodul und/oder mit einem Gehäuseelement der Vorrichtung aufweisen. Die Module sind über die genannten Schnittstellen einfach miteinander zu kombinieren und zu koppeln. So kann insbesondere einfach ein bestimmtes Modul eines Modulsatzes (Set aus Modulen), zum Beispiel ein Stützflächenmodul mit einem ersten Stützflächendurchmesser, gegen ein anders Modul des Modulsatzes, zum Beispiel gegen ein Stützflächenmodul mit einem zweiten Stützflächendurchmesser ausgewechselt werden, wobei die übrigen Module, also das Gehäusemodul und ein ggf. vorhandenes Montagemodul unverändert bleiben können.

Nach der Erfindung kann insbesondere eine Mehrzahl von Stützflächenmodulen zum Beispiel in Form eines Sets oder Modulsatzes, vorgesehen sein. Die einzelnen Module des Satzes können sich insbesondere jeweils hinsichtlich des Radius ihrer Stützfläche und/oder hinsichtlich des durch die Stützfläche ausgebildeten Umschlingungswinkels voneinander unterscheiden. In ähnlicher Weise kann nach der Erfindung eine Mehrzahl von Gehäusemodulen vorgesehen sein, die sich jeweils hinsichtlich bestimmter Eigenschaften, wie insbesondere Fluidleitungsaufnahme, Sensorbestückung, Deckelaufnahme und/oder Design, voneinander unterscheiden.

Es ist besonders vorteilhaft, wenn das Stützflächenmodul ein Strangpresselement oder Tiefziehelement ist oder durch Kaltumformen ausgebildet ist. Es kann insbesondere aus einem metallischen Werkstoff oder aus Kunststoff bestehen. Derartige Module sind einfach und kostengünstig herzustellen. Dieses Stützflächenmodul kann auch ein extrudiertes Kunststoff-Bauteil, ein Spritzgussteil oder ein mechanisch bearbeitetes Metallteil (Rohr, Platte, etc.) sein.

Nach einer Ausführungsform der Erfindung kann das Montagemodul als separates Bauteil ausgebildet sein. Alternativ kann es Bestandteil eines Gehäuses der Vorrichtung sein, insbesondere einer Gerätefront des Gerätegehäuses. Auf diese Weise ist eine erfindungsgemäße Peristaltikpumpe einfach mit bestehenden Vorrichtungen zur extrakorporalen Blutbehandlung verwendbar. Des Weiteren kann sie besonders einfach und effektiv von einem Maschinentyp entfernt und an einem anderen Maschinentyp angeordnet und verwendet werden.

Nach einer Ausführungsform ist die Stützfläche durch Kaltumformen, insbesondere durch Tiefziehen, in dem Maschinengehäuse, insbesondere in einem eine Maschinenfront ausbildenden Blechteil, ausgebildet. Die Ausbildung der Stützfläche kann so ohne Aufwand in einen üblichen Prozess zur Herstellung des Maschinengehäuses oder zumindest von Teilen davon integriert werden.

Nach einer Ausführungsform kann das Stützflächenmodul einer Pumpe ein Standard für eine Geräteplattform sein. Je nach Ausstattung der Maschine (zum Beispiel in einer hochwertigen "High End"-Version, in einer Standardversion oder als Budgetversion) kann dieses Stützflächenmodul mit unterschiedlichen Gehäusemodulen, die auch als Funktionsmodul Gehäuse bezeichnet werden können, zum Beispiel mit einer Sichtblende High End, einer Sichtblende Standard oder einer Sichtblende Budget ausgestattet oder kombiniert sein. Anders ausgedrückt kann die jeweilige Maschinenvariante mit einem Standard-Stützflächenmodul durch geeignete Kombination mit entsprechenden Gehäusemodulen (oder Montagemodulen) bestimmt sein. Dabei können die Funktionsmodule unterschiedlichste Zusatzfunktionen entsprechend den jeweiligen Ausstattungsmerkmalen aufweisen.

Man kann auch sagen, dass die Erfindung sich auf ein Gehäuse einer Blutpumpe bezieht, das Bestandteil einer peristaltisch arbeitenden Rollenpumpe, insbesondere einer Schlauchpumpe für die Medizintechnik ist. Die Pumpe kann ihren bestimmungsgemäßen Einsatz zum Beispiel insbesondere in der extrakorporalen Blutbehandlung finden. Ein Rotor ermöglicht zusammen mit elastischen Materialeigenschaften eines Pumpensegmentes eines Schlauchsystems, zum Beispiel in Form eines elastischen Schlauchs, eine Pumpfunktion, die beispielsweise eine Blutförderung zu einem Dialysator sicherstellt. Dabei kann das Pumpensegment des Schlauchsystems schlaufenförmig gegen eine Lauffläche oder Stützfläche eines Funktionsmoduls, das auch als Stützflächenmodul oder Laufflächenmodul bezeichnet werden kann, eingelegt werden. Die Stütz- oder Lauffläche kann dabei über ihren zylindrischen Durchmesser und ihren zylindrischen Umschlingungswinkel Einfluss auf die Menge des geförderten Mediums nehmen. Das Funktionsmodul "Stützfläche" kann aus unterschiedlichsten Materialien wie Metall oder Kunststoff und mittels unterschiedlichsten Fertigungsverfahren wie zum Beispiel Fräsen, Strangpressen, Tiefziehen, Druckguss, Extrudieren, Spritzguss etc. hergestellt sein. Das Funktionsmodul "Gehäuse" (Sichtblende) kann ebenfalls aus unterschiedlichsten Materialien wie Metall oder Kunststoff bestehen und mittels unterschiedlichster Fertigungsverfahren wie Fräsen, Strangpressen, Tiefziehen, Druckguss, Extrudieren, Spritzguss etc. hergestellt sein. Auch das Funktionsmodul "Montage" (Aufnahme) kann aus unterschiedlichsten Materialien wie Metall oder Kunststoff bestehen und mittels unterschiedlichster Fertigungsverfahren wie z.B. Fräsen, Strangpressen, Tiefziehen, Druckguss, Extrudieren, Spritzguss etc. hergestellt sein.

### Figurenbeschreibung

Die Erfindung wird im Folgenden anhand beispielhafter, nicht einschränkender und in den angehängten Figuren gezeigter Ausführungsformen näher erläutert. Dabei zeigt:
Figur 1 eine schematische Darstellung eines Ausschnitts einer Vorrichtung zur extrakorporalen Blutbehandlung,
Figur 2 eine schematische Darstellung eine Pumpengehäuses nach dem Stand der Technik,
Figur 3 eine schematische Aufsicht auf ein Stützflächenmodul einer erfindungsgemäßen Peristaltikpumpe,
Figur 4 das Stützflächenmodul der Figur 3 in einer perspektivischen Ansicht, und
Figur 5 das Stützflächenmodul der Figuren 3 und 4 mit einem damit kombinierbaren Gehäusemodul und einem damit kombinierbaren Montagemodul.

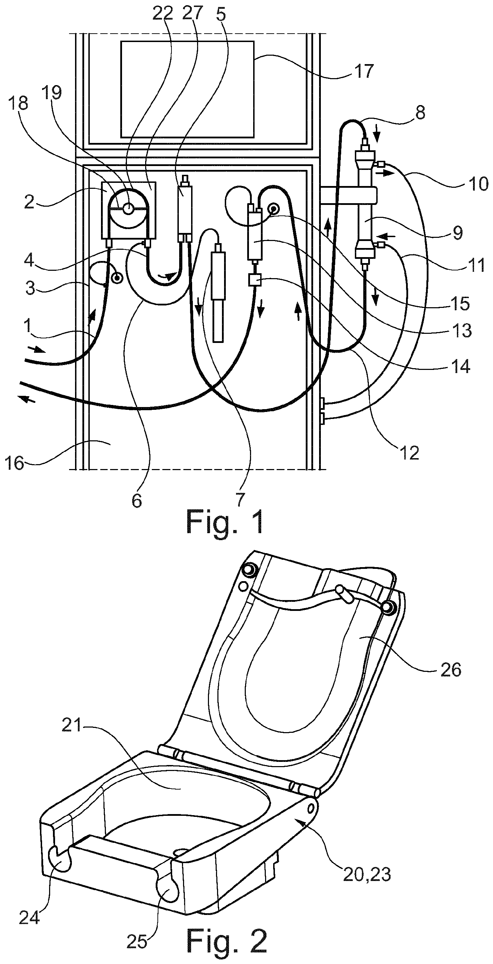

Figur 1 zeigt beispielhaft einen Ausschnitt einer Vorrichtung zur extrakorporalen Blutbehandlung nach der Erfindung. Gezeigt ist im Wesentlichen der gesamte extrakorporale Blutkreislauf der Vorrichtung. Dieser weist eine arterielle Blutleitung 1 auf, mittels der Blut von einem nicht gezeigten Patienten zu einer modular aufgebauten Peristaltikpumpe 2 der Behandlungsvorrichtung geführt wird. Vor der Peristaltikpumpe 2 ist ein arterieller Druckabnehmer 3 vorgesehen, mit dem der Druck vor der Peristaltikpumpe 2, also der niederdruckseitige Druck gemessen wird. Hochdruckseitig der Peristaltikpumpe 2 führt eine Hochdruckblutleitung 4 zu einem arteriellen Blutfänger 5. Unmittelbar am Ausgang der Peristaltikpumpe 2 kann mittels einer Zuleitung 6 und einer Pumpe 7 Zusatzstoff in das im System befindlichen Blut gegeben werden, z.B. Heparin zur Blutverdünnung.

Vom arteriellen Blutfänger 5 führt eine Leitung 8 unter Hochdruck stehendes aber noch unbehandeltes, mit Schlackenstoffen belastetes Blut zu einem Dialysator 9. Diesem wird eingangsseitig Dialysierflüssigkeit über eine Dialysierflüssigkeitszuleitung 10 zugeführt. Im Dialysator 9 wird Blut in bekannter Weise mittels der Dialysierflüssigkeit behandelt, z.B. gereinigt. Verbrauchte Dialysierflüssigkeit wird über eine Dialysierflüssigkeitsableitung 11 vom Dialysator 9 entfernt und einer nicht dargestellten Entsorgung oder Aufbereitung zugeführt. Behandeltes Blut wird mittels einer Blutableitung 12 vom Dialysator 9 zu einem venösen Luftfänger 13 geleitet, wo mittels einer Luftfalle 14 Luft abgeschieden wird. Am venösen Luftfänger 13 ist ein venöser Druckaufnehmer 15 vorgesehen, mit dem der venöse Druck, also der hochdruckseitige Druck, erfasst wird. Von der Luftfalle 14 wird behandeltes Blut über eine venöse Blutleitung 16 zurück zum Patienten geleitet. Dargestellt in Figur 1 ist auch eine Einheit 17 zur Überwachung und Steuerung der Vorrichtung. Die Vorrichtung zur extrakorporalen Blutbehandlung ist mit einem Gehäuse 100 gekapselt, das zumindest teilweise als Blechformteil ausgebildet ist.

Figur 2 zeigt ein Blutpumpengehäuse 20 nach dem Stand der Technik. Dieses ist als separates Aluminium-Frästeil 23 ausgebildet, das auf die Gehäusefront 100 des Gerätes montiert werden kann. Das Aluminium-Frästeil 23 ist verhältnismäßig komplex mit einer Einlassnut 24 und einer Auslassnut 25 für die Fluidleitung 22. Die Stützfläche 21 ist im Aluminium-Frästeil 23 durch eine eingefräste Vertiefung ausgebildet, was einen hohen Materialverbrauch zur Folge hat. Des Weiteren ist ein Deckel 26 nicht austauschbar am Gehäuse 20 angelenkt. Soll eine andere Stützflächenkontur oder ein anderer Deckel 26 verwendet werden, ist stets das gesamte Blutpumpengehäuse 20 auszutauschen.

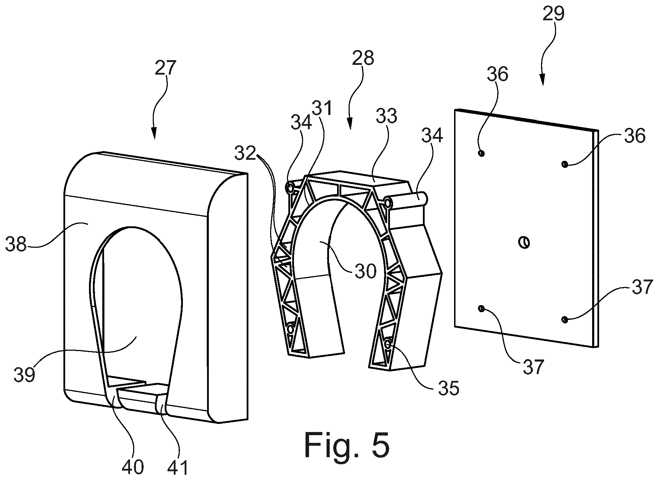

Die modulare Peristaltikpumpe 2 nach der Erfindung weist einen Rotor 18 auf, der um eine Rotorachse 19 rotiert. Die Peristaltikpumpe 2 weist des Weiteren ein in Figur 1 angedeutetes Gehäusemodul 27 auf. Das Gehäusemodul 27 ist zusammen mit einem damit kombinierbaren Stützflächenmodul 28 und einem Montagemodul 29 in Figur 5 in einer Art Explosionsansicht dargestellt. Das Gehäusemodul 27 bildet zusammen mit dem Stützflächenmodul 28 und dem Montagemodul 29 ein Pumpengehäuse für die Peristaltikpumpe 2 aus. Jedes der genannten drei Module 27, 28, 29 kann dabei jeweils zu einem Modulsatz mit jeweils unterschiedlichen Modulen 27, 28, 29 gehören. Diese Module können nach der Erfindung allesamt miteinander kombinierbar sein. Erreicht wird dies unter anderem dadurch, dass die Module 27, 28, 29 standardisierte Schnittstellen aufweisen, die eine gegenseitige Kopplung ermöglichen.

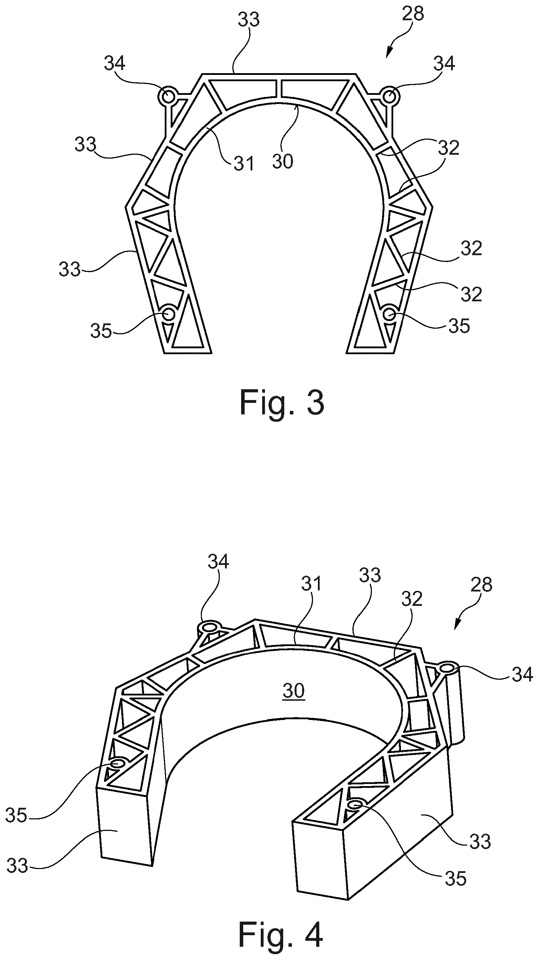

Die Schnittstelle des Stützflächenmoduls 28 ist besonders gut in den Figuren 3 und 4 zu erkennen. Das Stützflächenmodul 28 ist ein Strangpressteil mit einer integrierten Stützfläche 30. Diese ist an einer hufeisenförmig gebogenen Innenwandung 31 ausgebildet, die über Verstrebungen 32 mit einer ebenfalls gebogenen Außenwandung 33 verbunden ist. Außen an der Außenwandung 33 sind Aufnahmen 34 für nicht dargestellte Schrauben oder Bolzen ausgebildet. Zwischen den Verstrebungen sind zwei weitere Aufnahmen 35 für ebenfalls nicht dargestellte Schrauben oder Bolzen angeordnet. Die Aufnahmen 34, 35 bilden eine Schnittstelle aus, über die das Stützflächenmodul 28 mit dem Gehäusemodul 27 und/oder mit dem Montagemodul 29 kombinierbar und koppelbar ist. In einem Satz von Stützflächenmodulen 28 sind eine Mehrzahl von Stützflächenmodulen 28 enthalten, die sich zum Beispiel hinsichtlich der Krümmung der Stützfläche 30 voneinander unterscheiden. Die durch die Aufnahmen 34, 35 gebildeten Schnittstellen sind jedoch immer gleich, so dass jedes Stützstellenmodule 34, 35 des Satzes mit jedem Gehäusemodul (ggf. eines Satzes von Gehäusemodulen) und/oder Montagemodulen (ggf. eines Satzes von Montagemodulen) kombinierbar und koppelbar ist.

In Fig. 5 sind Schnittstellen ausbildende Aufnahmen 36, 37 des Montagemoduls 29 angedeutet. Die Schnittstellen des Gehäusemoduls 27 sind in Fig. 5 nicht zu erkennen, da sie von einer eine Abdeckung bildenden Vorderwandung 38 verdeckt sind. In dieser ist eine zentrale Ausnehmung 39 ausgebildet, über die der Rotor 18 sowie eine in den Figuren nicht dargestellte und zwischen Rotor 18 und Stützfläche 30 angeordnete elastisch verformbare Fluidleitung 22 zu erkennen sind. Fig. 5 zeigt angedeutet, dass das Gehäusemodul 27 als weitere Funktionseinheiten Aufnahmen 40, 41 für die Blutleitungen 1, 4 aufweist. Die Fluidleitung 22 wird bei Rotation des Rotors 18 deformiert. Sie ist eingangsseitig, also niederdruckseitig, mit der arteriellen Blutleitung 1 und ausgangsseitig, also hochdruckseitig, mit der Hochdruckblutleitung 4 verbunden. Sie wird zwischen dem Rotor 18 und der Stützfläche 21 derart deformiert, dass ihr Querschnitt im fehlerfreien Normalbetrieb der Pumpe vorzugsweise vollständig zusammengequetscht, also im Wesentlichen fluiddicht geschlossen wird.

## Patentansprüche

1. Peristaltikpumpe (2) einer Vorrichtung zur extrakorporalen Blutbehandlung, insbesondere einer Dialysemaschine, zum Fördern von Fluid in der Vorrichtung,
welche Peristaltikpumpe (2) einen um eine Rotorachse (19) rotierbar angetriebenen Rotor (18) und ein den Rotor (18) zumindest teilweise umgebendes Pumpengehäuse (27, 28, 29) mit einer um die Rotorachse (19) bogenförmig ausgebildeten Stützfläche (30) aufweist, die an einer hufeisenförmig gebogenen Innenwandung (31) ausgebildet ist, die über Verstrebungen (32) mit einer ebenfalls gebogenen Außenwandung (33) verbunden ist,
wobei eine elastisch verformbare Fluidleitung (22) zwischen dem Rotor (18) und der Stützfläche (30) positionierbar ist und bei zur Fluidförderung durch Rotation des Rotors (18) eine Querschnittsverengung ausbildend zwischen diesem und der Stützfläche (30) verformt wird, wobei
das Pumpengehäuse (27, 28, 29) modular ausgebildet ist und zumindest ein erstes, die Stützfläche (30) ausbildendes Stützflächenmodul (28) und zumindest ein zweites, mit diesem zu koppelndes und den Rotor (18) zumindest teilweise umgebendes oder aufnehmendes Gehäusemodul (27) aufweist, und
wobei das Stützflächenmodul (28) zumindest eine standardisierte Schnittstelle (34, 35) zum Koppeln mit dem Gehäusemodul (27) und/oder einem Montagemodul (29) und/oder mit einem Gehäuseelement der Vorrichtung aufweist.

2. Peristaltikpumpe (2) nach Anspruch 1, wobei das Pumpengehäuse (27, 28, 29) das Montagemodul (29) aufweist, das einerseits mit dem Stützflächenmodul (28) und/oder mit dem Gehäusemodul (27) und andererseits mit der Vorrichtung zur extrakorporalen Blutbehandlung, insbesondere mit einem Gehäuse der Vorrichtung, koppelbar ist.

3. Peristaltikpumpe (2) nach einem der vorstehenden Ansprüche, wobei das Gehäusemodul (27) eine standardisierte Schnittstelle (36, 37) zum Koppeln mit dem Stützflächenmodul (28) und/oder dem Montagemodul (29) und/oder mit einem Gehäuseelement der Vorrichtung aufweist.

4. Peristaltikpumpe (2) nach einem der Ansprüche 1 bis 3, wobei das Montagemodul (29) eine standardisierte Schnittstelle (36, 37) zum Koppeln mit dem Stützflächenmodul (28) und/oder dem Gehäusemodul (27) und/oder mit einem Gehäuseelement der Vorrichtung aufweist.

5. Peristaltikpumpe (2) nach einem der vorstehenden Ansprüche, wobei eine Mehrzahl von Stützflächenmodulen (28) vorgesehen ist, die sich jeweils hinsichtlich des Radius ihrer Stützfläche (30) und/oder hinsichtlich des durch die Stützfläche (30) ausgebildeten Umschlingungswinkels voneinander unterscheiden.

6. Peristaltikpumpe (2) nach einem der vorstehenden Ansprüche, wobei eine Mehrzahl von Gehäusemodulen (27) vorgesehen ist, die sich jeweils hinsichtlich bestimmter Eigenschaften wie insbesondere Fluidleitungsaufnahme (40, 41), Sensorbestückung, Deckelaufnahme und/oder Design voneinander unterscheiden.

7. Peristaltikpumpe (2) nach einem der vorstehenden Ansprüche, wobei das Stützflächenmodul (28) ein Strangpresselement oder Tiefziehelement ist oder durch Kaltumformen ausgebildet ist.

8. Peristaltikpumpe (2) nach einem der Ansprüche 1 bis 7, wobei das Stützflächenmodul (28) ein extrudiertes Kunststoff-Bauteil, ein Spritzgussbauteil oder ein mechanisch bearbeitetes Metallteil ist.

9. Peristaltikpumpe (2) nach einem der Ansprüche 1 bis 8, wobei das Montagemodul (29) als separates Bauteil ausgebildet ist oder Bestandteil eines Gehäuses der Vorrichtung ist.

10. Peristaltikpumpe (2) nach einem der vorstehenden Ansprüche, wobei das Stützflächenmodul (28) aus einem metallischen Werkstoff oder aus Kunststoff besteht.

11. Vorrichtung zur extrakorporalen Blutbehandlung mit einer Peristaltikpumpe (2) nach einem der vorstehenden Ansprüche.

## Claims

1. A peristaltic pump (2) of an apparatus for extracorporeal blood treatment,
especially of a dialysis machine, for delivering fluid in the apparatus,
said peristaltic pump (2) comprising a rotor (18) driven to rotate about a rotor axis (19) and a pump casing (27, 28, 29) surrounding the rotor (18) at least in part and having a supporting surface (30) which is configured to be curved around the rotor axis (19) and which is formed on an inner wall (31) bent in horse-shoe shape which is connected to a likewise bent outer wall (33) via braces (32),
wherein an elastically deformable fluid line (22) is adapted to be positioned between the rotor (18) and the supporting surface (30) and is deformed between the rotor (18) and the supporting surface (30) when forming a cross-sectional constriction for delivering fluid by rotation of the rotor (18),
**wherein**
the pump casing (27, 28, 29) has a modular design and includes at least a first supporting surface module (28) configuring the supporting surface (30) and at least a second casing module (27) to be coupled to the former and at least partly surrounding or receiving the rotor (18), and
wherein the supporting surface module (28) comprises at least one standardized interface (34, 35) for coupling to the casing module (27) and/or to a mounting module (29) and/or to a casing element of the apparatus.

2. The peristaltic pump (2) according to claim 1, wherein the pump casing (27, 28, 29) includes the mounting module (29) which can be coupled, on the one hand, to the supporting surface module (28) and/or to the casing module (27) and, on the other hand, to the apparatus for extracorporeal blood treatment, especially to a casing of the apparatus.

3. The peristaltic pump (2) according to one of the preceding claims, wherein the casing module (27) comprises a standardized interface (36, 37) for coupling to the supporting surface module (28) and/or the mounting module (29) and/or to a casing element of the apparatus.

4. The peristaltic pump (2) according to one of claims 1 to 3, wherein the mounting module (29) comprises a standardized interface (36, 37) for coupling to the supporting surface module (28) and/or the casing module (27) and/or to a casing element of the apparatus.

5. The peristaltic pump (2) according to one of the preceding claims, wherein a plurality of supporting surface modules (28) is provided which are different from each other as regards the radius of their supporting surface (30) and/or as regards the wrapping angle formed by the supporting surface (30).

6. The peristaltic pump (2) according to one of the preceding claims, wherein a plurality of casing modules (27) is provided which are different from each other as regards specific characteristics such as especially the fluid line adapter (40, 41), the equipment with sensors, the cover adapter and/or the design.

7. The peristaltic pump (2) according to one of the preceding claims, wherein the supporting surface module (28) is an extrusion-molded element or a deepdrawn element or is formed by cold forming.

8. The peristaltic pump (2) according to one of claims 1 to 7, wherein the supporting surface module (28) is an extruded plastic component, an injection-molded component or a mechanically machined metal part.

9. The peristaltic pump (2) according to one of claims 1 to 8, wherein the mounting module (29) is in the form of a separate component or is part of a casing of the apparatus.

10. The peristaltic pump (2) according to one of the preceding claims, wherein the supporting surface module (28) is made from a metallic material or from plastic material.

11. An apparatus for extracorporeal blood treatment comprising a peristaltic pump (2) according to one of the preceding claims.

## Revendications

1. Pompe péristaltique (2) d'un dispositif de traitement du sang extracorporel, en particulier d'une machine de dialyse, pour refouler du fluide dans le dispositif,
laquelle pompe péristaltique (2) présente un rotor (18) entraîné de manière à pouvoir tourner autour d'un axe de rotor (19) et un carter de pompe (27, 28, 29) entourant au moins en partie le rotor (18) avec une surface de support (30) réalisée en forme d'arc autour de l'axe de rotor (19), qui est réalisée sur une paroi intérieure (31) cintrée en forme de fer à cheval, qui est reliée à une paroi extérieure (33) également cintrée par l'intermédiaire d'entretoises (32),
dans laquelle un conduit de fluide (22) élastiquement déformable peut être positionné entre le rotor (18) et la surface de support (30) et est déformé pour le refoulement de fluide par rotation du rotor (18) en réalisant un rétrécissement de section transversale entre celui-ci et la surface de support (30), dans laquelle
le carter de pompe (27, 28, 29) est réalisé de manière modulaire et présente au moins un premier module de surface de support (28) réalisant la surface de support (30) et au moins un deuxième module de carter (27) à coupler à ce dernier et entourant ou recevant au moins en partie le rotor (18), et
dans laquelle le module de surface de support (28) présente au moins une interface (34, 35) standardisée destinée à être couplée au module de carter (27) et/ou à un module de montage (29) et/ou à un élément de carter du dispositif.

2. Pompe péristaltique (2) selon la revendication 1, dans laquelle le carter de pompe (27, 28, 29) présente le module de montage (29), qui peut être couplé d'une part au module de surface de support (28) et/ou au module de carter (27) et d'autre part au dispositif pour le traitement du sang extracorporel, en particulier à un carter du dispositif.

3. Pompe péristaltique (2) selon l'une quelconque des revendications précédentes, dans laquelle le module de carter (27) présente une interface (36, 37) standardisée destinée à être couplée au module de surface de support (28) et/ou au module de montage (29) et/ou à un élément de carter du dispositif.

4. Pompe péristaltique (2) selon l'une quelconque des revendications 1 à 3, dans laquelle le module de montage (29) présente une interface (36, 37) standardisée destinée à être couplée au module de surface de support (28) et/ou au module de carter (27) et/ou à un élément de carter du dispositif.

5. Pompe péristaltique (2) selon l'une quelconque des revendications précédentes, dans laquelle une multitude de modules de surface de support (28) est prévue, qui se distinguent les uns des autres respectivement eu égard au rayon de leur surface de support (30) et/ou eu égard à l'angle d'enroulement réalisé par la surface de support (30).

6. Pompe péristaltique (2) selon l'une quelconque des revendications précédentes, dans laquelle une multitude de modules de carter (27) est prévue, qui se distinguent les uns des autres respectivement eu égard à des propriétés définies telles qu'en particulier un logement de conduit de fluide (40, 41), un équipement en capteur, un logement de couvercle et/ou un design.

7. Pompe péristaltique (2) selon l'une quelconque des revendications précédentes, dans laquelle le module de surface de support (28) est un élément extrudé ou un élément d'emboutissage ou est réalisé par un façonnage à froid.

8. Pompe péristaltique (2) selon l'une quelconque des revendications 1 à 7, dans laquelle le module de surface de support (28) est un composant en matière plastique extrudé, un composant moulé par injection ou une pièce métallique usinée mécaniquement.

9. Pompe péristaltique (2) selon l'une quelconque des revendications 1 à 8, dans laquelle le module de montage (29) est réalisé en tant que composant séparé ou constituant d'un carter du dispositif.

10. Pompe péristaltique (2) selon l'une quelconque des revendications précédentes, dans laquelle le module de surface de support (28) est constitué d'un matériau métallique ou d'une matière plastique.

11. Dispositif de traitement du sang extracorporel avec une pompe péristaltique (2) selon l'une quelconque des revendications précédentes.
